# EUROPÄISCHE PATENTSCHRIFT

(11) **EP 1 713 804 B1**
(45) Veröffentlichungstag und Bekanntmachung des Hinweises auf die Patenterteilung: **19.03.2008**
(21) Anmeldenummer: 05700960.7
(22) Anmeldetag: 15.01.2005
(51) Int. Cl.: C07D 471/04, A61K 31/4375, A61P 3/10

(54) **CYCLOALKYL SUBSTITUIERTE 7-AMINO-4-CHINOLON-3-CARBONSÄURE-DERIVATE, VERFAHREN ZU IHRER HERSTELLUNG UND IHRE VERWENDUNG ALS ARZNEIMITTEL**
CYCLOALKYL SUBSTITUTED 7-AMINO-4-QUINOLONE-3-CARBOXYLIC ACID DERIVATIVES, METHOD FOR THE PRODUCTION THEREOF AND THEIR USE AS MEDICAMENTS
DERIVES D'ACIDE 7-AMINO-4-QUINOLONE-3-CARBOXILIQUE SUBSTITUES PAR CYCLOALKYLE, PROCEDE PERMETTANT DE LES PRODUIRE ET LEUR UTILISATION COMME MEDICAMENTS

(30) Priorität: 31.01.2004 DE 102004004971
(43) Veröffentlichungstag der Anmeldung: 25.10.2006
(73) Patentinhaber: Sanofi-Aventis Deutschland GmbH, 65929 Frankfurt am Main (DE)
(72) Erfinder: DEFOSSA, Elisabeth, 65510 Idstein (DE); KADEREIT, Dieter, 63071 Offenbach (DE); KLABUNDE, Thomas, 65929 Frankfurt (DE); SCHMOLL, Dieter, 65929 Frankfurt (DE); HERLING, Andreas, 65520 Bad Camberg (DE); WENDT, Karl-Ulrich, 65926 Frankfurt (DE); RUF, Sven, 65926 Frankfurt (DE)
(86) Internationale Anmeldenummer: PCT/EP2005/000373
(87) Internationale Veröffentlichungsnummer: WO 2005/073231

(56) Entgegenhaltungen:
- WO-A-03/074532

## Beschreibung

Die Erfindung betrifft Cycloalkyl substituierte 7-Amino-4-chinolon-3-carbonsäureDerivate sowie deren physiologisch verträgliche Salze und physiologisch funktionelle Derivate.

Es sind bereits strukturähnliche Verbindungen im Stand der Technik beschrieben (Link, Helmut; Bernauer, Karl; Englert Gerhard, Helvetica Chimica Acta 65(8), 1982, 2645-2667).

Der Erfindung lag die Aufgabe zugrunde, Verbindungen zur Verfügung zu stellen, die eine therapeutisch verwertbare Blutzucker senkende Wirkung entfalten. Hemmer der glykogenphosphorylase sind aus WO 03/74532 bekannt.

Die Erfindung betrifft daher Verbindungen der Formel I, 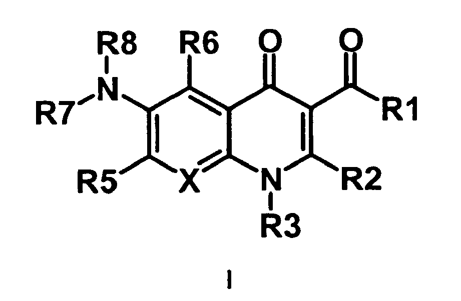 worin bedeuten
- R1: OH, O-(C₁-C₆)-Alkyl, NH₂, NH-(C₁-C₆)-Alkyl, N-((C₁-C₆)-Alkyl)₂ oder O-(C₁-C₆)-OCO-(C₁-C₆)-Alkyl;
- R2: H, (C₁-C₆)-Alkyl oder Phenyl;
- R3: H, (C₁-C₈)-Alkyl, (C₃-C₇)-Cycloalkyl, Pyridyl oder Phenyl, wobei Alkyl durch R9 und wobei Pyridyl oder Phenyl durch R10 substituiert sein können;
- R9: NH₂, NH-(C₁-C₆)-Alkyl, N-((C₁-C₆)-Alkyl)₂, -(C₃-C₇)-Cycloalkyl, Heteroalkyl, Heteroaryl, O-Phenyl oder Phenyl, wobei Phenyl oder Heteroaryl durch R11 substituiert sein können;
- R10: F, Cl, Br, (C₁-C₆-Alkyl), O-(C₁-C₆)-Alkyl, COOH, COO-(C₁-C₆)-Alkyl, NH₂, NH-(C₁-C₆)-Alkyl oder N-((C₁-C₆)-Alkyl)₂;
- R11: F, Cl, (C₁-C₆-Alkyl), O-(C₁-C₆)-Alkyl, COOH oder COO-(C₁-C₄)-alkyl;
- X: N;
- R5, R6: unabhängig voneinander H, F, Cl, Br, OH, NO₂, CN, (C₁-C₆)-Alkyl oder O-(C₁-C₆)-Alkyl, wobei Alkyl mehrfach durch F, Cl oder Br substituiert sein kann;
- R7: H oder (C₁-C₆)-Alkyl;
- R8: (C₃-C₁₂)-Cycloalkyl, wobei Cycloalkyl mit (C₁-C₄)-Alkyl, (C₁-C₄)-Alkenyl, (C₁-C₄)-Alkinyl, F, Cl, CN, CF₃, COOH oder COO-(C₁-C₄)-Alkyl substituiert sein kann;
sowie deren physiologisch verträgliche Salze.

Bevorzugt sind Verbindungen der Formel I, worin ein oder mehrere Reste die folgende Bedeutung haben:
- R1: OH, O-(C₁-C₆)-Alkyl, NH₂, NH-(C₁-C₆)-Alkyl, N-((C₁-C₆)-Alkyl)₂ oder O-(C₁-C₆)-OCO-(C₁-C₆)-Alkyl;
- R2: H;
- R3: H, (C₁-C₈)-Alkyl, (C₃-C₇)-Cycloalkyl, Pyridyl oder Phenyl, wobei Alkyl durch R9 und wobei Pyridyl oder Phenyl durch R10 substituiert sein können;
- R9: NH₂, NH-(C₁-C₆)-Alkyl, N-((C₁-C₆)-Alkyl)₂, -(C₃-C₇)-Cycloalkyl, Heteroalkyl, Heteroaryl, O-Phenyl oder Phenyl, wobei Phenyl oder Heteroaryl durch R11 substituiert sein können;
- R10: F, Cl, Br, (C₁-C₆-Alkyl), O-(C₁-C₆)-Alkyl, COOH, COO-(C₁-C₆)-Alkyl, NH₂, NH-(C₁-C₆)-Alkyl oder N-((C₁-C₆)-Alkyl)₂;
- R11: F, Cl, (C₁-C₆-Alkyl), O-(C₁-C₆)-Alkyl, COOH oder COO-(C₁-C₄)-alkyl;
- X: N;
- R5: H, F, Cl, Br, OH, NO₂, CN, (C₁-C₆)-Alkyl oder O-(C₁-C₆)-Alkyl, wobei Alkyl mehrfach durch F, Cl oder Br substituiert sein kann;
- R6: H;
- R7: H;
- R8: (C₃-C₁₂)-Cycloalkyl, wobei Cycloalkyl mit (C₁-C₄)-Alkyl, (C₁-C₄)-Alkenyl, (C₁-C₄)-Alkinyl, F, Cl, CN, CF₃, COOH oder COO-(C₁-C₄)-Alkyl substituiert sein kann;
sowie deren physiologisch verträgliche Salze.

Besonders bevorzugt sind Verbindungen der Formel I, worin ein oder mehrere Reste die folgende Bedeutung haben:
- R1: OH, O-(C₁-C₆)-Alkyl,;
- R2: H;
- R3: (C₁-C₈)-Alkyl,;
- R9: NH₂, NH-(C₁-C₆)-Alkyl, N-((C₁-C₆)-Alkyl)₂, -(C₃-C₇)-Cycloalkyl, Heteroalkyl, Heteroaryl, O-Phenyl oder Phenyl, wobei Phenyl oder Heteroaryl durch R11 substituiert sein können;
- R10: F, Cl, Br, (C₁-C₆-Alkyl), O-(C₁-C₆)-Alkyl, COOH, COO-(C₁-C₆)-Alkyl, NH₂, NH-(C₁-C₆)-Alkyl oder N-((C₁-C₆)-Alkyl)₂;
- R11: F, Cl, (C₁-C₆-Alkyl), O-(C₁-C₆)-Alkyl, COOH oder COO-(C₁-C₄)-alkyl;
- X: N;
- R5: H, F, Cl, Br, OH, NO₂, CN, (C₁-C₆)-Alkyl oder O-(C₁-C₆)-Alkyl, wobei Alkyl mehrfach durch F, Cl oder Br substituiert sein kann;
- R6: H;
- R7: H;
- R8: (C₃-C₁₂)-Cycloalkyl, wobei Cycloalkyl mit (C₁-C₄)-Alkyl, (C₁-C₄)-Alkenyl, (C₁-C₄)-Alkinyl, F, Cl, CN, CF₃, COOH oder COO-(C₁-C₄)-Alkyl substituiert sein kann;
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Die Alkylreste in den Substituenten R1, R2, R3, R5, R6, R7, R8, R9, R10 und R11 können sowohl geradkettig wie verzweigt sein.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin, oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Heteroarylrest wird ein Pyrrolyl-, Pyridinyl-, Pyrimidinyl-, Pyrazinyl-, Indolyl-, Benzimidazolyl- Thiophenyl- oder ein Furanylrest verstanden.

Die Verbindung(en) der Formel (I) können auch in Kombination mit weiteren Wirkstoff verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag.

Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP -Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/US 11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide , BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure-{4-[(4-amino-quinazolin-2-ylamino)-methyl]-cyclohexylmethyl}-amid Hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1 H-indol-6-yloxy)-ethylamino]-ethanol Hydrochlorid (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5),230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

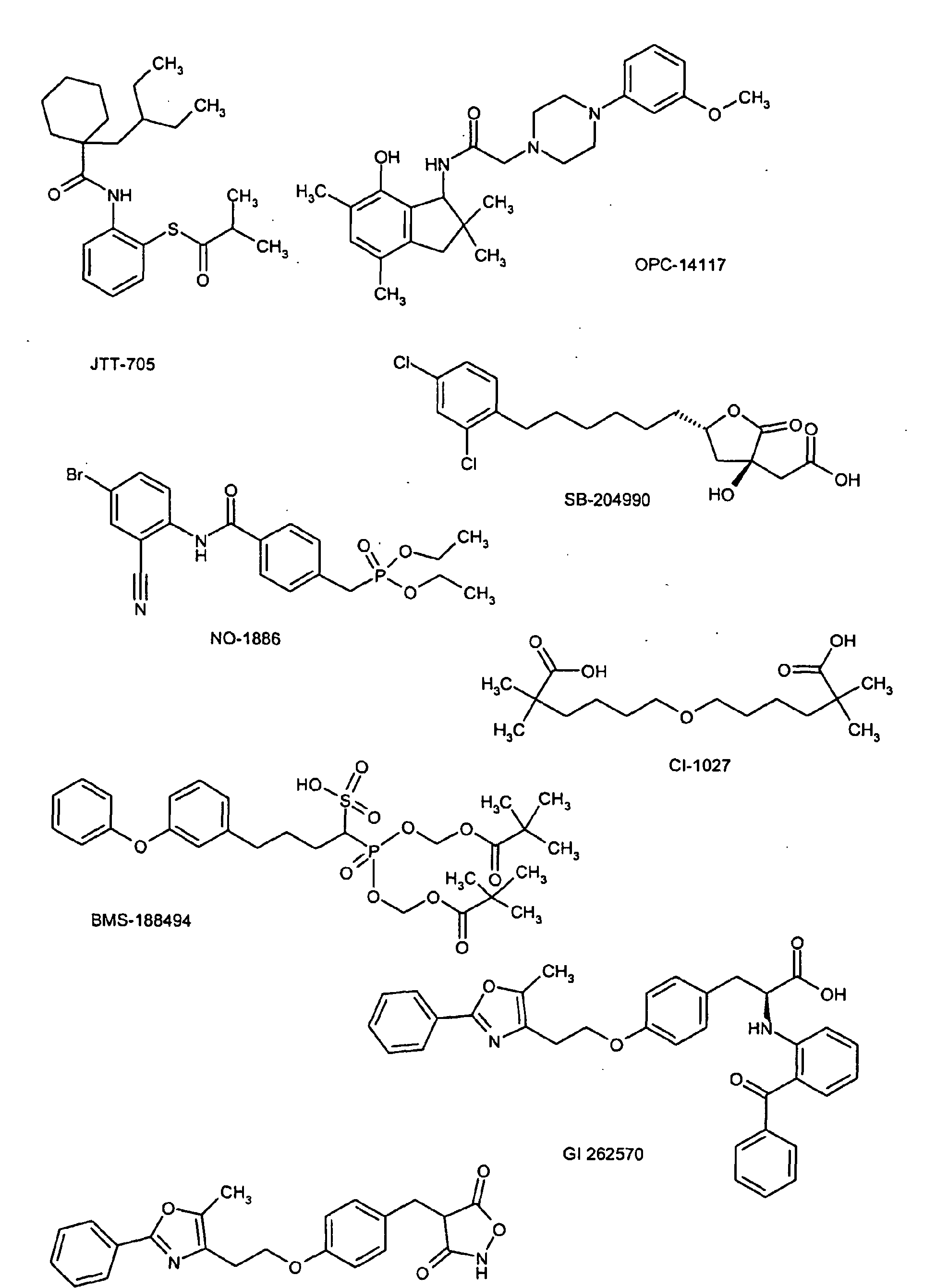

Die Herstellung der Verbindungen der Formel I ist in den folgenden Schemata beschrieben:
Verbindungen der Formel II können unter Buchwald Bedingungen mit Aminen der Formel III zu den Verbindungen der Formel IV, in denen R1' die Bedeutung eines Esters hat, umgesetzt werden. Y steht dabei für Br, I oder Triflat Bei diesen Buchwaldbedingungen können Katalysatorsysteme mit Pd(OAc)₂ oder Pd₂(dba)₃ als Palladium-Quellen, BINAP, Xanthphos und DPPF als Liganden und Cs₂CO₃, K₃PO₄ oder NaO^{t}Bu als Basen eingesetzt werden. Als Lösungsmittel kann beispielsweise Toluol, DME, Dioxan, THF oder DMF Verwendung finden. Die Reaktionsbedingungen können unter herkömmlicher Erwärmung oder Erwärmung und Reaktion in der Mikrowelle gewählt werden. (Literatur: Buchwald, Acc. Chem. Res. 1998, 31, 805) Eventuelle anschließende Verseifung der Verbindungen der Formel IV und eventuelle Umsetzung zu verschiedenen Amiden oder Estern führt zu Verbindungen der Formel I.

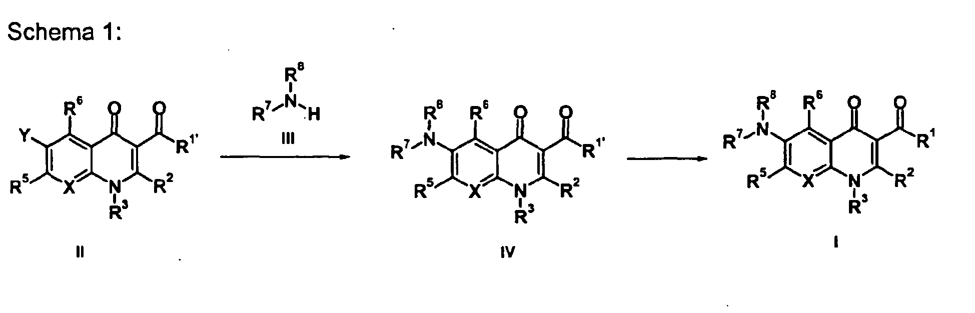

Die nachfolgend aufgeführten Beispiele dienen zur Erläuterung der Erfindung, ohne diese jedoch einzuschränken.

**Tabelle 1:**

| | | | | | | | | |
|---|---|---|---|---|---|---|---|---|
| 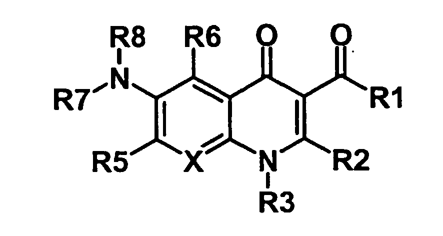 | | | | | | | | |

| **Beispiel** | **R1** | **R2** | **R3** | **X** | **R5** | **R6** | **R7** | **R8** |
|---|---|---|---|---|---|---|---|---|
| **1** | OEt | H | Et | N | Me | H | H | 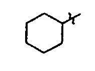 |
| **2** | OEt | H | Et | N | Me | H | H | 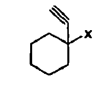 |
| **3** | OH | H | Et | N | Me | H | H | 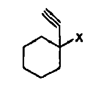 |
| **4** | OH | H | Et | N | Me | H | H | 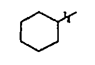 |

Die Wirksamkeit der Verbindungen wurde wie folgt getestet:

### Glykogenphosphorylase a Aktivitätstest

Der Effekt von Verbindungen auf die Aktivität der aktiven Form der Glykogenphosphorylase (GPa) wurde in der umgekehrten Richtung, durch Verfolgen der Glykogensynthese aus Glukose-1-Phosphat an Hand der Bestimmung der Freisetzung von anorganischem Phosphat, gemessen. Alle Reaktionen wurden als Doppelbestimmungen in Mikrotiterplatten mit 96-Vertiefungen (Half Area Plates, Costar Nr. 3696) durchgeführt, wobei die Änderung der Absorption auf Grund der Bildung des Reaktionsprodukts bei der weiter unten spezifizierten Wellenlänge in einem Multiskan Ascent Elisa Reader (Lab Systems, Finnland) gemessen wurde. Um die GPa Enzymaktivität in der umgekehrten Richtung zu messen, wurde die Umwandlung von Glukose-1-Phosphat in Glykogen und anorganisches Phosphat nach der allgemeinen Methode von Engers et al. (Engers HD, Shechosky S, Madsen NB, Can J Biochem 1970 Jul;48(7):746-754) mit folgenden Modifikationen gemessen: Humane Glykogenphosphorylase a (zum Beispiel mit 0,76 mg Protein / ml (Aventis Pharma Deutschland GmbH), gelöst in Pufferlösung E (25 mM β-Glyzerophosphat, pH 7,0, 1 mM EDTA und 1 mM Dithiotreitol) wurde mit Puffer T (50 mM Hepes, pH 7,0, 100 mM KCI, 2,5 mM EDTA, 2,5 mM MgCl₂·6H₂O) und Zusatz von 5 mg/ml Glykogen auf eine Konzentration von 10 µg Protein/ml verdünnt. Prüfsubstanzen wurden als 10 mM Lösung in DMSO zubereitet und auf 50 µM mit Pufferlösung T verdünnt. Zu 10 µl dieser Lösung wurden 10 µl 37,5 mM Glukose, gelöst in Pufferlösung T und 5 mg/mL Glykogen, sowie 10 µl einer Lösung von humaner Glykogenphosphorylase a (10 µg Protein/ml) und 20 µl Glukose-1-Phosphat, 2,5 mM zugegeben. Der basale Wert der Glykogenphosphorylase a Aktivität in Abwesenheit von Prüfsubstanz wurde durch Zugabe von 10 µl Pufferlösung T (0,1 % DMSO) bestimmt. Die Mischung wurde 40 Minuten bei Raumtemperatur inkubiert und das freigesetzte anorganische Phosphat mittels der allgemeinen Methode von Drueckes et al. (Drueckes P, Schinzel R, Palm D, Anal Biochem 1995 Sep 1;230(1):173-177) mit folgenden Modifikationen gemessen: 50 µl einer Stop-Lösung von 7,3 mM Ammoniummolybdat, 10,9 mM Zinkacetat, 3,6 % Askorbinsäure, 0,9 % SDS werden zu 50 µl der Enzymmischung gegeben. Nach 60 Minuten Inkubation bei 45 °C wurde die Absorption bei 820 nm gemessen. Zur Bestimmung der Hintergrundsabsorption wurde in einem separaten Ansatz die Stop-Lösung unmittelbar nach Zugabe der Glukose-1-Phosphatlösung zugegeben.
Dieser Test wurde mit einer Konzentrationen von 10 µM der Prüfsubstanz durchgeführt, um die jeweilige Hemmung der Glykogenphosphorylase a in vitro durch die Prüfsubstanz zu bestimmen.

**Tabelle 2: Biologische Aktivität**

| **Bsp.** | **% Hemmung bei 10 µM** |
|---|---|
| **1** | 24 |
| **2** | 42 |
| **3** | 35 |
| **4** | 16 |

Aus der Tabelle ist abzulesen, dass die Verbindungen der Formel I die Aktivität der Glykogenphosphorylase a hemmen und dadurch zur Senkung des Blutzuckerspiegels gut geeignet sind.

Nachfolgend wird die Herstellung einiger Beispiele detailliert beschrieben, die übrigen Verbindungen der Formel I wurden analog erhalten:
Experimenteller Teil:

### Beispiel 2:

### 1-Ethyl-6-(1-ethynyl-cyclohexylamino)-7-methyl-4-oxo-1,4-dihydro-[1,8]naphthyridine-3-carbonsäureethylester

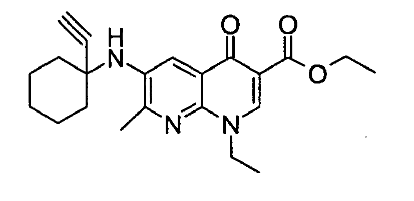

100 mg 6-Bromo-1-ethyl-8-methyl-4-oxo-1,4-dihydro-chinolon-3-carbonsäuremethylester wurden zusammen mit 36 mg 1-Ethynyl-cyclohexylamine, 6,6 mg Pd(OAc)₂, 36 mg XANTPHOS und 200 mg Cäsiumkarbonat in ein geeignetes Reaktionsgefäß überführt, man erzeugte mit Argon eine Schutzgasatmosphäre und versetzte mit 10 ml Dioxan. Anschließend erhitzte man für 8 Stunden auf 80°C. Das reine Produkt wurde aus der Reaktionslösung durch Chromatographie auf einer HPLC-Anlage isoliert. Dabei wurde eine Merck Purospher-RP18 Säule sowie ein Acetonitril:Wasser-Gemisch als Eluent verwendet, der Anfangsgehalt an Acetonitril betrug 15 % und stieg innerhalb von 20 Minuten auf 95 %.
Ausbeute: 4 %

### Beispiel 3:

### 1-Ethyl-6-(1-ethynyl-cyclohexylamino)-7-methyl-4-oxo-1,4-dihydro-[1,8]naphthyridine-3-carbonsäure

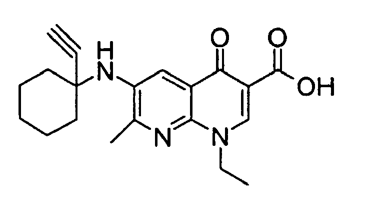

Man löste den 1-Ethyl-6-(1-ethynyl-cyclohexylamino)-7-methyl-4-oxo-1,4-dihydro-[1,8]naphthyridine-3-carbonsäureethylester (10 mg) in 5 ml Dioxan, versetzte mit 2,5 Äquivalenten einer 1 N NaOH-Lösung und erhitzte für 4 Stunden auf 60°C. Nach der Entfernung des Lösungsmittels im Vakuum erfolgte zur Reinigung des Produktes eine Chromatographie auf einer HPLC-Anlage. Das reine Produkt wurde aus der Reaktionslösung durch Chromatographie auf einer HPLC-Anlage isoliert. Dabei wurde eine Merck Purospher-RP18 Säule sowie ein Acetonitril:Wasser-Gemisch als Eluent verwendet, der Anfangsgehalt an Acetonitril betrug 15 % und stieg innerhalb von 20 Minuten auf 95 %.
Ausbeute: 85%

## Patentansprüche

1. Verbindungen der Formel I, 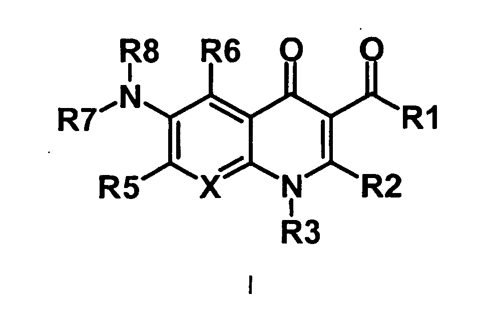 worin bedeuten
R1 OH, O-(C₁-C₆)-Alkyl, NH₂, NH-(C₁-C₆)-Alkyl, N-((C₁-C₆)-Alkyl)₂ oder O-(C₁-C₆)-OCO-(C₁-C₆)-Alkyl;
R2 H, (C₁-C₆)-Alkyl oder Phenyl;
R3 H, (C₁-C₈)-Alkyl, (C₃-C₇)-Cycloalkyl, Pyridyl oder Phenyl, wobei Alkyl durch R9 und wobei Pyridyl oder Phenyl durch R10 substituiert sein können;
R9 NH₂, NH-(C₁-C₆)-Alkyl, N-((C₁-C₆)-Alkyl)₂, -(C₃-C₇)-Cycloalkyl, Heteroalkyl, Heteroaryl, O-Phenyl oder Phenyl, wobei Phenyl und Heteroaryl durch R11 substituiert sein können;
R10 F, Cl, Br, (C₁-C₆-Alkyl), O-(C₁-C₆)-Alkyl, COOH, COO-(C₁-C₆)-Alkyl, NH₂, NH-(C₁-C₆)-Alkyl oder N-((C₁-C₆)-Alkyl)₂;
R11 F, Cl, (C₁-C₆-Alkyl), O-(C₁-C₆)-Alkyl, COOH oder COO-(C₁-C₄)-alkyl;
X N;
R5, R6 unabhängig voneinander H, F, Cl, Br, OH, NO₂, CN, (C₁-C₆)-Alkyl oder O-(C₁-C₆)-Alkyl, wobei Alkyl mehrfach durch F, Cl oder Br substituiert sein kann;
R7 H oder (C₁-C₆)-Alkyl;
R8 (C₃-C₁₂)-Cycloalkyl, wobei Cycloalkyl mit (C₁-C₄)-Alkyl, (C₁-C₄)-Alkenyl, (C₁-C₄)-Alkinyl,, F, Cl, CN, CF₃, COOH oder COO-(C₁-C₄)-alkyl substituiert sein kann;
sowie deren physiologisch verträgliche Salze.

2. Verbindungen der Formel I, gemäß Anspruch 1, **dadurch gekennzeichnet, daß** darin bedeuten
R1 OH, O-(C₁-C₆)-Alkyl, NH₂, NH-(C₁-C₆)-Alkyl, N-((C₁-C₆)-Alkyl)₂ oder O-(C₁-C₆)-OCO-(C₁-C₆)-Alkyl;
R2 H;
R3 H, (C₁-C₈)-Alkyl, (C₃-C₇)-Cycloalkyl, Pyridyl oder Phenyl, wobei Alkyl durch R9 und wobei Pyridyl oder Phenyl durch R10 substituiert sein können;
R9 NH₂, NH-(C₁-C₆)-Alkyl, N-((C₁-C₆)-Alkyl)₂, -(C₃-C₇)-Cycloalkyl, Heteroalkyl, Heteroaryl, O-Phenyl oder Phenyl, wobei Phenyl oder Heteroaryl durch R11 substituiert sein können;
R10 F, Cl, Br, (C₁-C₆-Alkyl), O-(C₁-C₆)-Alkyl, COOH, COO-(C₁-C₆)-Alkyl, NH₂, NH-(C₁-C₆)-Alkyl oder N-((C₁-C₆)-Alkyl)₂;
R11 F, Cl, (C₁-C₆-Alkyl), O-(C₁-C₆)-Alkyl, COOH oder COO-(C₁-C₄)-alkyl;
X N;
R5 H, F, Cl, Br, OH, NO₂, CN, (C₁-C₆)-Alkyl oder O-(C₁-C₆)-Alkyl, wobei Alkyl mehrfach durch F, Cl oder Br substituiert sein kann;
R6 H;
R7 H;
R8 (C₃-C₁₂)-Cycloalkyl, wobei Cycloalkyl mit (C₁-C₄)-Alkyl, (C₁-C₄)-Alkenyl, (C₁-C₄)-Alkinyl, F, Cl, CN, CF₃, COOH oder COO-(C₁-C₄)-Alkyl substituiert sein kann;
sowie deren physiologisch verträgliche Salze.

3. Verbindungen der Formel I, gemäß Anspruch 1 oder 2, **dadurch gekennzeichnet, daß** darin bedeuten
R1 OH, O-(C₁-C₆)-Alkyl, ;
R2 H;
R3 (C₁-C₈)-Alkyl,;
R9 NH₂, NH-(C₁-C₆)-Alkyl, N-((C₁-C₆)-Alkyl)₂, -(C₃-C₇)-Cycloalkyl, Heteroalkyl, Heteroaryl, O-Phenyl oder Phenyl, wobei Phenyl oder Heteroaryl durch R11 substituiert sein können;
R10 F, Cl, Br, (C₁-C₆-Alkyl), O-(C₁-C₆)-Alkyl, COOH, COO-(C₁-C₆)-Alkyl, NH₂, NH-(C₁-C₆)-Alkyl oder N-((C₁-C₆)-Alkyl)₂;
R11 F, Cl, (C₁-C₆-Alkyl), O-(C₁-C₆)-Alkyl, COOH oder COO-(C₁-C₄)-alkyl;
X N;
R5 H, F, Cl, Br, OH, NO₂, CN, (C₁-C₆)-Alkyl oder O-(C₁-C₆)-Alkyl, wobei Alkyl mehrfach durch F, Cl oder Br substituiert sein kann;
R6 H;
R7 H;
R8 (C₃-C₁₂)-Cycloalkyl, wobei Cycloalkyl mit (C₁-C₄)-Alkyl, (C₁-C₄)-Alkenyl, (C₁-C₄)-Alkinyl, F, Cl, CN, CF₃, COOH oder COO-(C₁-C₄)-Alkyl substituiert sein kann;
sowie deren physiologisch verträgliche Salze.

4. Verbindungen gemäß Anspruch 1 zur Anwendung als Arzneimittel.

5. Arzneimittel enthaltend eine oder mehrere der Verbindungen gemäß Anspruch 1.

6. Arzneimittel enthaltend eine oder mehrere der Verbindungen gemäß Anspruch 1 und mindestens einen weiteren Wirkstoff.

7. Arzneimittel, gemäß Anspruch 4, **dadurch gekennzeichnet, daß** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

8. Verwendung der Verbindungen gemäß Anspruch 1 zur Herstellung eines Medikamentes zur Blutzuckersenkung.

9. Verwendung der Verbindungen gemäß Anspruch 1 zur Herstellung eines Medikamentes zur Behandlung des Typ II Diabetes.

10. Verwendung der Verbindungen gemäß Anspruch 1 zur Herstellung eines Medikamentes zur Behandlung von Lipid- und Kohlenhydratstoffwechselstörungen.

11. Verwendung der Verbindungen gemäß Anspruch 1 zur Herstellung eines Medikaments zur Behandlung arteriosklerotischer Erscheinungen.

12. Verwendung der Verbindungen gemäß gemäß Anspruch 1 zur Herstellung eines Medikaments zur Behandlung von Insulin Resistenz.

13. Verfahren zur Herstellung eines Arzneimittels enthaltend eine oder mehrere der Verbindungen gemäß Anspruch 1, **dadurch gekennzeichnet, daß** der Wirkstoff mit einem pharmazeutisch geeigneten Träger vermischt wird und diese Mischung in eine für die Verabreichung geeignete Form gebracht wird.

## Claims

1. A compound of the formula I 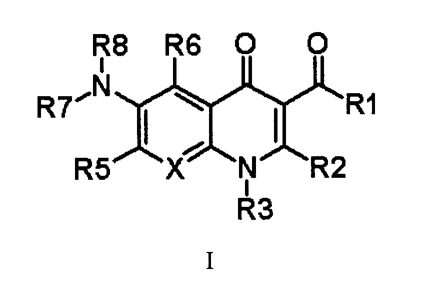 in which the meanings are
R1 OH, O-(C₁-C₆)-alkyl, NH₂, NH-(C₁-C₆)-alkyl, N-((C₁-C₆)-alkyl)₂ or O-(C₁-C₆)-OCO-(C₁-C₆)-alkyl;
R2 H, (C₁-C₆)-alkyl or phenyl;
R3 H, (C₁-C₈)-alkyl, (C₃-C₇)-cycloalkyl, pyridyl or phenyl, where alkyl may be substituted by R9 and where pyridyl or phenyl may be substituted by R10;
R9 NH₂, NH-(C₁-C₆)-alkyl, N-((C₁-C₆)-alkyl)₂, -(C₃-C₇)-cycloalkyl, heteroalkyl, heteroaryl, O-phenyl or phenyl, where phenyl and heteroaryl may be substituted by R11 ;
R10 F, Cl, Br, (C₁-C₆-alkyl), O-(C₁-C₆)-alkyl, COOH, COO-(C₁-C₆)-alkyl, NH₂, NH-(C₁-C₆)-alkyl or N-((C₁-C₆)-alkyl)₂;
R11 F, Cl, (C₁-C₆-alkyl), O-(C₁-C₆)-alkyl, COOH or COO-(C₁-C₄)-alkyl;
X N;
R5, R6 independently of one another H, F, Cl, Br, OH, NO₂, CN, (C₁-C₆)-alkyl or O-(C₁-C₆)-alkyl, where alkyl may be substituted more than once by F, Cl or Br;
R7 H or (C₁-C₆)-alkyl;
R8 (C₃-C₁₂)-cycloalkyl, where cycloalkyl may be substituted by (C₁-C₄)-alkyl, (C₁-C₄)-alkenyl, (C₁-C₄)-alkynyl, F, Cl, CN, CF₃, COOH or COO-(C₁-C₄)-alkyl;
and the physiologically tolerated salts thereof.

2. A compound of the formula I as claimed in claim 1 in which the meanings are
R1 OH, O-(C₁-C₆)-alkyl, NH₂, NH-(C₁-C₆)-alkyl, N-((C₁-C₆)-alkyl)₂ or O-(C₁-C₆)-OCO-(C₁-C₆)-alkyl;
R2 H;
R3 H, (C₁-C₈)-alkyl, (C₃-C₇)-cycloalkyl, pyridyl or phenyl, where alkyl may be substituted by R9 and where pyridyl or phenyl may be substituted by R10;
R9 NH₂, NH-(C₁-C₆)-alkyl, N-((C₁-C₆)-alkyl)₂, -(C₃-C₇)-cycloalkyl, heteroalkyl, heteroaryl, O-phenyl or phenyl, where phenyl or heteroaryl may be substituted by R11 ;
R10 F, Cl, Br, (C₁-C₆-alkyl), O-(C₁-C₆)-alkyl, COOH, COO-(C₁-C₆)-alkyl, NH₂, NH-(C₁-C₆)-alkyl or N-((C₁-C₆)-alkyl)₂;
R11 F, Cl, (C₁-C₆-alkyl), O-(C₁-C₆)-alkyl, COOH or COO-(C₁-C₄)-alkyl;
X N;
R5 H, F, Cl, Br, OH, NO₂, CN, (C₁-C₆)-alkyl or O-(C₁-C₆)-alkyl, where alkyl may be substituted more than once by F, Cl or Br;
R6 H;
R7 H;
R8 (C₃-C₁₂)-cycloalkyl, where cycloalkyl may be substituted by (C₁-C₄)-alkyl, (C₁-C₄)-alkenyl, (C₁-C₄)-alkynyl, F, Cl, CN, CF₃, COOH or COO-(C₁-C₄)-alkyl;
and the physiologically tolerated salts thereof.

3. A compound of the formula I as claimed in claim 1 or 2 in which the meanings are
R1 OH, O-(C₁-C₆)-alkyl;
R2 H;
R3 (C₁-C₈)-alkyl;
R9 NH₂, NH-(C₁-C₆)-alkyl, N-((C₁-C₆)-alkyl)₂, -(C₃-C₇)-cycloalkyl, heteroalkyl, heteroaryl, O-phenyl or phenyl, where phenyl or heteroaryl may be substituted by R11;
R10 F, Cl, Br, (C₁-C₆-alkyl), O-(C₁-C₆)-alkyl, COOH, COO-(C₁-C₆)-alkyl, NH₂, NH-(C₁-C₆)-alkyl or N-((C₁-C₆)-alkyl)₂;
R11 F, Cl, (C₁-C₆-alkyl), O-(C₁-C₆)-alkyl, COOH or COO-(C₁-C₄)-alkyl;
X N;
R5 H, F, Cl, Br, OH, NO₂, CN, (C₁-C₆)-alkyl or O-(C₁-C₆)-alkyl, where alkyl may be substituted more than once by F, Cl or Br;
R6 H;
R7 H;
R8 (C₃-C₁₂)-cycloalkyl, where cycloalkyl may be substituted by (C₁-C₄)-alkyl, (C₁-C₄)-alkenyl, (C₁-C₄)-alkynyl, F, Cl, CN, CF₃, COOH or COO-(C₁-C₄)-alkyl;
and the physiologically tolerated salts thereof.

4. A compound as claimed in claim 1 for use as medicament.

5. A medicament comprising one or more of the compounds as claimed in claim 1.

6. A medicament comprising one or more of the compounds as claimed in claim 1 and at least one other active ingredient.

7. The medicament as claimed in claim 4, wherein the other active ingredient comprises one or more
antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyte-stimulating hormone) agonists, CCK agonists, serotonin reuptake inhibitors, mixed sertoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

8. The use of the compounds as claimed in claim 1 for producing a medicament for reducing blood glucose.

9. The use of the compounds as claimed in claim 1 for producing a medicament for the treatment of type II diabetes.

10. The use of the compounds as claimed in claim 1 for producing a medicament for the treatment of disturbances of lipid and carbohydrate metabolism.

11. The use of the compounds as claimed in claim 1 for producing a medicament for the treatment of arteriosclerotic manifestations.

12. The use of the compounds as claimed in claim 1 for producing a medicament for the treatment of insulin resistance.

13. A process for producing a medicament comprising one or more of the compounds as claimed in claim 1, which comprises mixing the active ingredient with a pharmaceutically suitable carrier and converting this mixture into a form suitable for administration.

## Revendications

1. Composés de formule I, 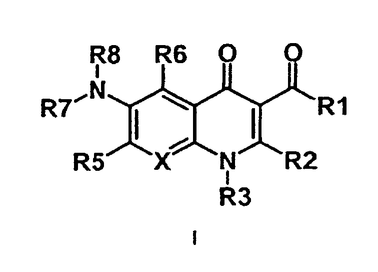 dans laquelle
R1 représente un groupe OH, un groupe O-(C₁-C₆)-alkyle, un groupe NH2, un groupe NH- (C₁-C₆) -alkyle, un groupe N- ( (C₁-C₆) -alkyl)₂ ou un groupe O- (C₁-C₆) -OCO- (C₁-C₆) -alkyle ;
R2 représente un atome d'hydrogène, un groupe alkyle en (C₁-C₆) ou un groupe phényle ;
R3 représente un atome d'hydrogène, un groupe alkyle en (C₁-C₈), un groupe cycloalkyle en (C₃-C₇), un groupe pyridyle ou un groupe phényle, où un groupe alkyle peut être substitué par R9 et où un groupe pyridyle ou un groupe phényle peut être substitué par R10 ;
R9 représente un groupe NH₂, un groupe NH-(C₁-C₆) -alkyle, un groupe N-((C₁-C₆)-alkyl)₂, un groupe cyclo-alkyle en (C₃-C₇), un groupe hétéroalkyle, un groupe hétéroaryle, un groupe 0-phényle ou un groupe phényle, où un groupe phényle et un groupe hétéroaryle peuvent être substitués par R11 ;
R10 représente un atome de fluor, un atome de chlore, un atome de brome, un groupe alkyle en (C₁-C₆), un groupe O- (C₁-C₆)-alkyle, un groupe COOH, un groupe COO-(C₁-C₆)-alkyle, un groupe NH₂, un groupe NH-(C₁-C₆)-alkyle ou un groupe N-((C₁-C₆)-alkyl)₂ ;
R11 représente un atome de fluor, un atome de chlore, un groupe alkyle en (C₁-C₆), un groupe O-(C₁-C₆)-alkyle, un groupe COOH ou un groupe COO-(C₁-C₄)-alkyle;
X représente un atome d'azote ;
R5, R6 représentent, indépendamment les uns des autres, un atome d'hydrogène, un atome de fluor, un atome de chlore, un atome de brome, un groupe OH, un groupe NO₂, un groupe CN, un groupe alkyle en (C₁-C₆) ou un groupe O-(C₁-C₆)-alkyle, où un groupe alkyle peut être substitué plusieurs fois par un atome de fluor, un atome de chlore ou un atome de brome ;
R7 représente un atome d'hydrogène ou un groupe alkyle en (C₁-C₆) ;
R8 représente un groupe cycloalkyle en (C₃-C₁₂), où cycloalkyle peut être substitué par un groupe alkyle en (C₁-C₄), un groupe alcényle en (C₁-C₄) , un groupe alcynyle en (C₁-C₄) , un atome de fluor, un atome de chlore, un groupe CN, un groupe CF₃, un groupe COOH ou un groupe COO-(C₁-C₄)-alkyle ;
ainsi que leurs sels physiologiquement acceptables.

2. Composés de formule I, selon la revendication 1,
**caractérisés en ce que**
R1 représente un groupe OH, un groupe O- (C₁-C₆) -alkyle, un groupe NH₂, un groupe NH-(C₁-C₆)-alkyle, un groupe N-((C₁-C₆)-alkyl)₂ ou un groupe O-(C₁-C₆)-OCO-(C₁-C₆)-alkyle ;
R2 représente un atome d'hydrogène ;
R3 représente un atome d'hydrogène, un groupe alkyle en (C₁-C₈), un groupe cycloalkyle en (C₃-C₇), un groupe pyridyle ou un groupe phényle, où un groupe alkyle peut être substitué par R9 et où un groupe pyridyle ou un groupe phényle peut être substitué par R10 ;
R9 représente un groupe NH₂, un groupe NH- (C₁-C₆) -alkyle, un groupe N- ((C₁-C₆)-alkyl)₂, un groupe cyclo-alkyle en (C₃-C₇), un groupe hétéroalkyle, un groupe hétéroaryle, un groupe O-phényle ou un groupe phényle, où un groupe phényle ou un groupe hétéroaryle peut être substitués par R11 ;
R10 représente un atome de fluor, un atome de chlore, un atome de brome, un groupe alkyle en (C₁-C₆), un groupe O-(C₁-C₆)-alkyle, un groupe COOH, un groupe COO-(C₁-C₆)-alkyle, un groupe NH₂, un groupe NH-(C₁-C₆)-alkyle ou un groupe N-((C₁-C₆)-alkyl)₂ ;
R11 représente un atome de fluor, un atome de chlore, un groupe alkyle en (C₁-C₆), un groupe O-(C₁-C₆)-alkyle, un groupe COOH ou un groupe COO-(C₁-C₄)-alkyle ;
X représente un atome d'azote ;
R5 représente un atome d'hydrogène, un atome de fluor, un atome de chlore, un atome de brome, un groupe OH, un groupe NO₂, un groupe CN, un groupe alkyle en (C₁-C₆) ou un groupe O-(C₁-C₆)-alkyle, où un groupe alkyle peut être substitué plusieurs fois par un atome de fluor, un atome de chlore ou un atome de brome ;
R6 représente un atome d'hydrogène ;
R7 représente un atome d'hydrogène ;
R8 représente un groupe cycloalkyle en (C₃-C₁₂), où cycloalkyle peut être substitué par un groupe alkyle en (C₁-C₄), un groupe alcényle en (C₁-C₄), un groupe alcynyle en (C₁-C₄), un atome de fluor, un atome de chlore, un groupe CN, un groupe CF₃, un groupe COOH ou un groupe COO-(C₁-C₄)-alkyle ;
ainsi que leurs sels physiologiquement acceptables.

3. Composés de formule I, selon la revendication 1 ou 2, **caractérisés en ce que**
R1 représente un groupe OH, un groupe O-(C₁-C₆)-alkyle ;
R2 représente un atome d'hydrogène ;
R3 représente un groupe alkyle én (C₁-C₈) ;
R9 représente un groupe NH₂, un groupe NH- (C₁-C₆) -alkyle, un groupe N-((C₁-C₆)-alkyl)₂, un groupe cyclo-alkyle en (C₃-C₇), un groupe hétéroalkyle, un groupe hétéroaryle, un groupe 0-phényle ou un groupe phényle, où un groupe phényle ou un groupe hétéroaryle peut être substitués par R11 ;
R10 représente un atome de fluor, un atome de chlore, un atome de brome, un groupe alkyle en (C₁-C₆), un groupe O-(C₁-C₆)-alkyle, un groupe COOH, un groupe COO- (C₁-C₆) -alkyle, un groupe NH₂, un groupe NH-(C₁-C₆)-alkyle ou un groupe N- ((C₁-C₆) -alkyl) ₂ ;
R11 représente un atome de fluor, un atome de chlore, un groupe alkyle en (C₁-C₆), un groupe O-(C₁-C₆)-alkyle, un groupe COOH ou un groupe COO-(C₁-C₄)-alkyle ;
X représente un atome d'azote ;
R5 représente un atome d'hydrogène, un atome de fluor, un atome de chlore, un atome de brome, un groupe OH, un groupe NO₂, un groupe CN, un groupe alkyle en (C₁-C₆) ou un groupe O-(C₁-C₆)-alkyle, où un groupe alkyle peut être substitué plusieurs fois par un atome de fluor, un atome de chlore ou un atome de brome ;
R6 représente un atome d'hydrogène ;
R7 représente un atome d'hydrogène ;
R8 représente un groupe cycloalkyle en (C₃-C₁₂), où cycloalkyle peut être substitué par un groupe alkyle en (C₁-C₄), un groupe alcényle en (C₁-C₄), un groupe alcynyle en (C₁-C₄), un atome de fluor, un atome de chlore, un groupe CN, un groupe CF₃, un groupe COOH ou un groupe COO- (C₁-C₄) -alkyle ;
ainsi que leurs sels physiologiquement acceptables.

4. Composés selon la revendication 1 pour une application en tant que médicament.

5. Médicament comprenant un ou plusieurs des composés selon la revendication 1.

6. Médicament comprenant un ou plusieurs des composés selon la revendication 1 et au moins un ingrédient actif supplémentaire.

7. Médicament selon la revendication 4, **caractérisé en ce qu'**il comprend, en tant qu'ingrédient actif supplémentaire, un ou plusieurs :
agents antidiabétiques, ingrédients actifs hypoglycémiants, inhibiteurs de HMGCoA-réductase, inhibiteurs de la résorption du cholestérol, agonistes de PPAR gamma, agonistes de PPAR alpha, agonistes de PPAR alpha/gamma, fibrates, inhibiteurs de MTP, inhibiteurs de la résorption d'acide biliaire, inhibiteurs de CETP, adsorbeurs polymères d'acide biliaire, inducteurs de récepteur de LDL, inhibiteurs d'ACAT, antioxydants, inhibiteurs de lipoprotéine lipase, inhibiteurs d'ATP-citrate-lyase, inhibiteurs de squalène synthétase, antagonistes de la lipoprotéine (a), inhibiteurs de lipase, insulines, sulfonylurées, biguanides, méglitinides, thiazolidindiones, inhibiteurs de α-glucosidase, ingrédients actifs agissant sur le canal potassique ATP-dépendant des cellules bêta, agonistes de CART, agonistes de NPY, agonistes de MC4, agonistes de l'orexine, agonistes de H3, agonistes de TNF, agonistes de CRF, antagonistes de CRF BP, agonistes de l'urocortine, agonistes de β3, agonistes de MSH (hormone stimulant les mélanocytes), agonistes de CCK, inhibiteurs de la recapture de la sérotonine, composés mixtes sérotoninergiques et noradrénergiques, agonistes de 5HT, agonistes de la bombésine, antagonistes de la galanine, hormones de croissance, composés libérant une hormone de croissance, agonistes de TRH, modulateurs découpleurs de la protéine 2 ou 3, agonistes de la leptine, agonistes de DA (bromocriptine, doprexine), inhibiteurs de lipase/amylase, modulateurs de PPAR, modulateurs de RXR ou agonistes de TR-β ou amphétamines.

8. Utilisation des composés selon la revendication 1 pour la production d'un médicament destiné à réduire la glycémie.

9. Utilisation des composés selon la revendication 1 pour la production d'un médicament destiné au traitement du diabète du type II.

10. Utilisation des composés selon la revendication 1 pour la production d'un médicament destiné au traitement de troubles du métabolisme lipidique et glucidique.

11. Utilisation des composés selon la revendication 1 pour la production d'un médicament destiné au traitement de symptômes artériosclérotiques.

12. Utilisation des composés selon la revendication 1 pour la production d'un médicament destiné au traitement de la résistance à l'insuline.

13. Procédé de production d'un médicament comprenant un ou plusieurs des composés selon la revendication 1, **caractérisé en ce que** l'ingrédient actif est mélangé avec un support pharmaceutiquement approprié et ce mélange est mis sous une forme appropriée pour l'administration.
